# EUROPEAN PATENT SPECIFICATION

(11) **EP 3 090 777 B1**
(45) Date of publication and mention of the grant of the patent: **04.11.2020**
(21) Application number: 16175325.6
(22) Date of filing: 02.07.2013
(51) Int. Cl.: A61M 39/08, B32B 27/30, B32B 27/40, B32B 1/08

(54) **MULTI-LAYERED TUBING**
MEHRSCHICHTIGER SCHLAUCH
TUBE MULTICOUCHE

(30) Priority: 15.08.2012 US 201213586288; 27.11.2012 US 201213686197
(43) Date of publication of application: 09.11.2016
(62) Divisional of application: 13744861.9
(73) Proprietor: Tekni-Plex, Inc., King of Prussia, PA 19406 (US)
(72) Inventor: BOURGEOIS, Philip, Perrysburg, OH Ohio 43551 (US)
(74) Representative: Jenkins, Peter David

(56) References cited:
- WO-A1-03/064909
- WO-A1-2013/109329
- US-A1- 2009 087 606

## Description

### Field of the Invention

The present invention relates to polymeric tubing typically formed by a co-extrusion process, the tubing having multiple layers of different polymeric materials.

### Background

Tubing comprised of polymeric material is used in many industrial and commercial applications, including in the medical field. Various FDA compliant plastics are used, depending upon properties desired and the intended applications. Where the tubing is used to transport fluids for in vivo treatment of human patients or for delivery of any fluid where the fluid itself or a component contained in the fluid needs to be isolated and contained within and not leach through the walls of the tube, selection of a polymeric material having a selective property can be a factor.

Polyvinyl chloride (PVC) is one of the most widely used plastics. While structurally stable and easily formable into desired shapes, PVC is typically manufactured using plasticizers which can migrate out of the PVC matrix into bodily fluids and has other properties not ideally suited for medical treatment applications. Likewise, due to the inherent nature of plasticized PVC tubing, there arises the potential absorption of medicines and other components of aqueous fluids used in medical treatments into the sidewall of the PVC tube. U.S. Pat. No. 4,627,844 to Schmitt ("Schmitt") discloses a tri-layer tube which is embodied in a commercial product sold under the trademark "SUREPATH 151" by the Natvar Division of Tekni-Plex, Inc. As disclosed in Schmitt, an outer layer of PVC and an inner fluid-contact layer of low density polyethylene (LDPE) are co-extruded with an intermediate tie layer of ethylene vinyl acetate copolymer (EVA). Polyurethane is potentially a substitute for PVC as disclosed in commonly owned and co-pending U.S. Serial Nos. 13/354,029 and 13/586,288. Other tubing configurations are disclosed in U.S. Patent No. 7,647,949, U.S. Patent No. 4,211,741 and U.S. Patent Publication No. 2007/0119511.

### Summary of the Invention

The invention provides a tube according to claim 1 and a method of delivering a non-aqueous fluid according to claim 9. Preferred or optional features are defined in the dependent claims. In accordance with the preferred embodiments of the invention there is provided a tubing, tube or tubular device that comprises at least three concentric layers of polymeric materials comprising inner and outer layers of a polymeric material comprised of thermoplastic polyurethane and a middle layer comprised of a polymeric material comprised of an acrylate containing polymer that is disposed between and binds the inner and outer layers together by adhesion mechanisms, such as chemical adhesion, without the need for additional binders or adhesive layers. The polyurethane of which each of the inner and outer layers are comprised can be the same or different in structure, molecular weight, crystallinity, purity, monomeric unit content, branching, chain length, additive content, inorganic content and physical properties such as elongation, melting point, resistance to creep, and hardness.

The layers of polymeric materials are co-extruded together to form the tubing such that the outer and inner layers are adhered to the middle layer and thus all three layers adhered to each other. The tubing is formed with a central hollow channel, bore or passage that is radially surrounded and defined by the polymeric layers that act as the wall or walls of the tubing.

The tube or tubing is particularly suitable for transport of aqueous fluids such as fluids used in medical treatments and applications. Alternatively the tube or tubing can be used for routing, delivery and transport of liquid and gaseous non-aqueous and aqueous fluids including organic materials such as ethers, alcohols, hydrocarbons, amines, aldehydes, ketones, amides, carboxylic acids and esters and non-aqueous fluids such as oxygen, carbon dioxide, carbon monoxide, helium, neon, argon, krypton, xenon, radon, hydrogen, nitrogen and the like and mixtures of two or more of any of the foregoing with each other or one or more of the foregoing together with an aqueous fluid.

The polymeric material of the inner and outer layer is comprised of more than about 90% by weight of an aromatic or aliphatic polyether based polyurethane thermoplastic elastomeric material ("TPU") and the middle layer is comprised of an ethylene ethyl acrylate copolymer (EEA), ethylene methyl acrylate copolymer (EMA), an anhydride grafted ethylene methyl acrylate copolymer (AEMA), a copolymer of two or more of said acrylates or a mixture of two or more of the foregoing.

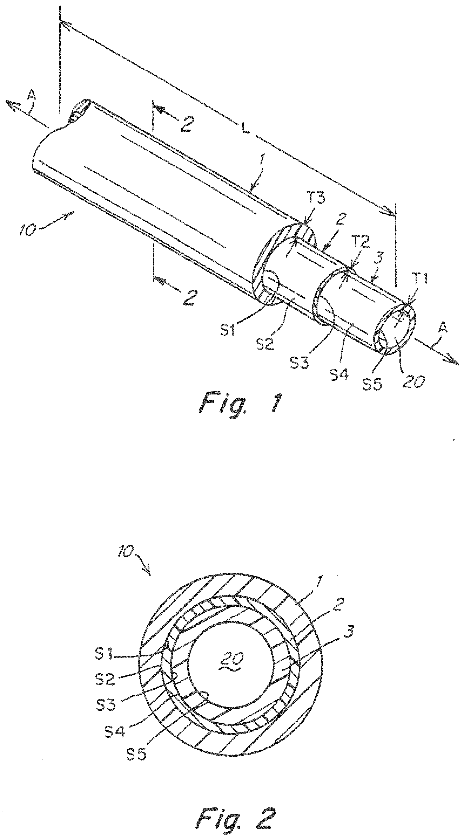

In one embodiment, the middle layer preferably has a thickness substantially greater than the combined thicknesses of the inner and outer layers. The cross-sectional thickness T2 of the middle layer, Fig. 1, typically ranges between about 5% and about 98% and more typically between about 10% and about 85% of the total cross-sectional wall thickness of the tube 10 (namely the thickness of the inner layer T1 plus the thickness of the middle layer T2 plus the thickness of the outer layer T3). The thickness T2 of the middle layer can range from about 5% to about 30% of the total cross-sectional thickness of the tube wall, or in another embodiment from about 31% to about 60%, and in yet another preferred embodiment from about 61% to about 98%. By increasing the thickness of the middle layer across the tube wall, and thus the proportional volume of the polymeric material of the middle layer of the tube vs. the volume of polymeric material of the inner and outer layers, a much lower cost and/or other benefit(s) can be obtained. For example, where the polymeric material of the middle layer is a soft ethylene ethyl acrylate (EEA) and/or ethylene methyl acrylate (EMA), these materials provide good adhesion to a thermoplastic polyurethane elastomer (TPU) and maintain resistance to delamination of the tube under high stress and temperature conditions, but are less costly than the thermoplastic polyurethane elastomers of which the inner and outer layers are comprised. Generally, the total wall thickness (T1 plus T2) of the inner and outer layers of thermoplastic polyurethane should be at least about 2 % of the total wall thickness of the tube (T1 plus T2 plus T3) for tubes greater than or equal to 2.54 mm (0.100 inches) in total wall thickness, at least about 3% of the total wall thickness for tubes greater than 1.295 mm 0.051 inches in total wall thickness but below 2.54 mm (0.100 inches) in total wall thickness and at least 5% of the total wall thickness for tubes greater than 0.254 mm (0.010 inches) but equal to or below 1.27 mm (0.050 inches) in total wall thickness in order to provide the necessary softness and elasticity along with mechanical properties required in a typical tubing application.

Thus, it is envisioned that in one embodiment the majority of the volume of the sidewall of a tube will be made from EEA or EMA material and the TPU inner layer and outer layer will be as thin as possible so as to still function as both the fluid contact layer (inner layer) and as the outer layer to which various fitments may be bonded (luers, etc.).

With reference to Figs. 1, 2, in one embodiment a tube 10 having a length L extending along a longitudinal tube axis A has inner and outer polyurethane layers 3, 1 which are between about 0.00635 mm (.00025 inches) and about 0.635 mm (.025 inches) in thickness, T1, T3, and a middle acrylate copolymer layer 2 of between about 0.203 mm (.008 inches) and about 3.048 mm (0.120 inches) in thickness, T2. The layers 1, 2, 3 collectively form a tubular wall surrounding and defining a central fluid flow passage 20.

Ethylene ethyl acrylate (EEA) copolymers, ethylene methyl acrylate (EMA) copolymers and anhydride grafted ethylene methyl acrylate (AEMA) copolymers are elastomeric in nature and have excellent visual clarity. In a typical co-extrusion process, TPU and EEA or EMA or AEMA are melt extruded through a die head to form a tubular shaped extrudate that is then cooled through conventional water baths or water vacuum tanks and which are either subsequently wound or cut into a particular length for use. The level of elasticity and softness of the EEA, EMA, AEMA or copolymer thereof is controlled through the amount of ethyl acrylate or methyl acrylate comonomer utilized with ethylene in the copolymerization process. The resulting three layer tubes manufactured by such a co-extrusion process act in a monolithic manner in that they return to close to their original shape and dimensions after being strained or stretched in a tensile manner along the longitudinal axis A of the tube at a stress of up to about 55 MPa and a strain of up to about 900-950 % and without any visual delamination between any of the layers after being submersed in water at about 60° C for about 36 hours.

The polymeric materials are preferably "contaminant free" meaning that they do not contain more than insignificant amounts of potentially unwanted materials (typically less than about out 0.5% and preferably less than about 0.2%, by weight) and/or prevent leaching or leaking of unwanted materials such as plasticizers, catalysts, monomers, metals, salts, ions or other substances that are potentially unwanted to a human being into an aqueous solution or other fluid medium with which one or the other of the three layers may come into contact during the normal course of use of the tubing in delivering aqueous (or non-aqueous) fluid, such as insulin, chemotherapy drugs and other potentially unstable aqueous drug suspensions, to or from a human subject. The middle layer prevents delamination of the outer and inner layers from the middle layer under conditions of relatively low to moderate stress or strain. In addition, the middle layer acts as a barrier to leaching or leaking of contaminants from the outer layer to or through the inner layer into the hollow central bore or passage of the tube.

In accordance with one aspect of the invention there is provided a tube comprising an inner layer, an outer layer and a middle layer, wherein the inner layer comprises a thermoplastic polyurethane, the outer layer comprises a thermoplastic polyurethane and the middle layer comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

The inner and outer layers each comprise more than about 90% by weight of an aromatic or aliphatic polyether based polyurethane. The middle layer can comprise more than about 90% by weight of an ethylene ethyl acrylate copolymer, an ethylene methyl acrlylate copolymer, an anhydride grafted ethylene methyl acrylate copolymer, or more than about 90% by weight of a mixture or copolymer of two or more of the foregoing.

One or both of the inner and outer layers can comprise a polytetramethyleneglycol-based polyurethane.

The ethylene methyl acrylate copolymer typically comprises at least about 19.5 percent methyl acrylate content by weight.

The ethylene ethyl acrylate copolymer typically comprises at least about 19.5 percent ethyl acrylate content by weight.

Also disclosed herein is a medical tube for transport of aqueous fluid comprising: an inner layer comprising more than about 90% by weight of a thermoplastic polyurethane, an outer layer comprising more than about 90% by weight of a thermoplastic polyurethane and, a middle layer disposed between the outer and inner layers comprising more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

Also disclosed herein is a tube for transport of a non-aqueous fluid comprising: an inner layer comprising more than about 90% by weight of a thermoplastic polyurethane, an outer layer comprising more than about 90% by weight of a thermoplastic polyurethane and, a middle layer disposed between the outer and inner layers comprising more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing. In such an embodiment the non-aqueous fluid can comprise a liquid or gaseous fluid including organic materials such as ethers, alcohols, hydrocarbons, amines, aldehydes, ketones, amides, carboxylic acids and esters and non-aqueous fluids such as oxygen, carbon dioxide, carbon monoxide, helium, neon, argon, krypton, xenon, radon, hydrogen, nitrogen and the like and mixtures of two or more of any of the foregoing with each other or one or more of the foregoing together with an aqueous fluid.

Also disclosed herein is a medical tube for transport of an aqueous fluid comprising: an inner layer comprising more than about 90% by weight of a thermoplastic polyurethane, an outer layer comprising more than about 90% by weight of a thermoplastic polyurethane and, a middle layer disposed between the inner and outer layers comprised of at least about 90% by weight of an ethylene ethyl acrylate copolymer, an ethylene methyl acrylate copolymer, an anhydride grafted ethylene methyl acrylate copolymer, a copolymer of two or more of said acrylates or a mixture of two or more of the foregoing, wherein the tube does not visually delaminate after being submersed in water at 60 °C for 36 hours.

Also disclosed herein is a medical tube for transport of an aqueous fluid comprising: an inner layer comprising more than about 90% by weight of a thermoplastic polyurethane, an outer layer comprising more than about 90% by weight of a thermoplastic polyurethane and, a middle layer comprised of at least about 90% by weight of an ethylene ethyl acrylate copolymer, an ethylene methyl acrylate copolymer, an anhydride grafted ethylene methyl acrylate copolymer, a copolymer of said acrylates or a mixture of two or more of the foregoing, wherein the tube does not visually delaminate at a stress of up to about 55 MPa and a strain of up to about 900-950 %, and, wherein the tube does not visually delaminate after being submersed in water at 60° C for 36 hours.

Also disclosed herein is a method of forming a medical tube comprising an outer layer, an inner layer and a middle layer disposed between the outer layer and the inner layer, the method comprising: selecting first and second polymeric materials each comprising more than about 90% by weight of a thermoplastic polyurethane; selecting a third polymeric material that readily bonds and adheres to the first and second polymeric materials on co-extrusion and cooling of the materials; co-extruding the selected first, second and third polymeric materials to form the medical tubing in a configuration such that the inner and outer layers comprise the first polymeric material and the middle layer comprises at least about 90% by weight of the second polymeric material. In such a method the first polymeric material preferably comprises more than about 90% by weight of an aromatic or aliphatic polyether based polyurethane.

Also disclosed herein is a method of forming a fluid delivery tube comprising;
selecting a first material comprised of more than about 90% by weight of a thermoplastic polyurethane,
selecting a second material comprised of more than about 90% by weight of a thermoplastic polyurethane,
selecting a third material comprised of more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing,
forming a tube comprised of an inner layer of the first material, an outer layer of the second material and a middle layer of the third material,
wherein the middle layer adheres the inner and outer layers together;
wherein the tube has a central fluid flow passage surrounded by the inner, middle and outer layers.

In such a method the step of forming typically comprises co-extruding the first, second and third materials simultaneously to form the inner and outer layers having a cross-sectional thickness of between about 0.00635 mm (.00025 inches) and 0.635 mm (about .025 inches) and to form the middle layer having a cross-sectional thickness of between about 0.203 mm (.008 inches) and about 3.048 mm (0.120 inches).

The step of forming can comprise co-extruding the first, second and third materials such that the middle layer is between about 5% and about 98% of the total cross-sectional thickness of the inner, outer and middle layers combined.

The step of forming can comprise co-extruding the first, second and third materials such that the middle layer is between about 10% and about 85% of the total cross-sectional thickness of the inner, outer and middle layers combined.

The step of forming can comprise co-extruding the first, second and third materials such that the middle layer is between about 61% and about 98% of the total cross-sectional thickness of the inner, outer and middle layers combined.

Also disclosed herein is a method of delivering an aqueous fluid to a subject comprising; selecting a tube comprising an inner layer, an outer layer and a middle layer, wherein the inner and outer layers comprise more than about 90% by weight of one or more thermoplastic polyurethanes and the middle layer comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing; wherein the tube has a central fluid flow passage surrounded by the layers; routing an aqueous fluid through the central fluid flow passage of the tube, and, delivering the aqueous fluid routed through the central fluid flow passage into a blood vessel of the subject. In such a method the step of selecting preferably comprises co-extruding the outer, inner and middle layers to form the tube such that the intermediate layer comprises at least about 90% by weight of one or more of the acrylate copolymers.

In an embodiment of the invention, such a method enables routing and delivery of liquid and gaseous non-aqueous fluids including organic materials such as ethers, alcohols, hydrocarbons, amines, aldehydes, ketones, amides, carboxylic acids, esters and the like and fluid materials such as oxygen, carbon dioxide, carbon monoxide, helium, neon, argon, krypton, xenon, radon, hydrogen, nitrogen and the like as well as mixtures of two or more of all of the foregoing with each other or mixtures of one or more of the foregoing together with an aqueous fluid.

Most preferably, the inner, middle and outer layers of the aforementioned tubes or tubing do not visually delaminate from each other at a stress up to of about 55 MPa and a strain of up to about 900-950% when measured by pulling a length of tubing about 50.8 mm (2 inches( in axial length along its axis using a Lloyd LR5K plus mechanical tester at a pull rate of about 0.3048 m/minute (12 inches/minute) at ambient environmental conditions of about 22 degrees C (72 degrees F) and about 50% relative humidity, the break point of the tubing 10 being about 57-62 MPa and about 1000-1050%.

Most preferably, all of the aforementioned tubes or medical tubing do not visually delaminate after being subjected to submersion in water at 60° C for 36 hours and subsequently mechanically flattened by manual squeezing of the tube from its normal round in cross-sectional condition to a flattened or oval shape cross-sectional shape or condition.

Preferably the aforementioned tubes or medical tubing have a central axial fluid flow passage through which aqueous fluid is routed, the inner layer having a radially inner wall surface that contacts the aqueous fluid, the outer and inner layers resisting delamination from the middle layer at a stress of up to about 55 MPa and a strain of up to about 900-950%.

Most preferably the middle layer serves as a barrier against, prevents or substantially lessens migration of mobile moieties such as monomers, short chained polymers, ions, water, small organic molecules, metals, plasticizers, catalysts and the like between the outer and inner layers or from the outer layer into the inner layer or the central flow passage or from the central flow passage or inner layer into the outer layer.

In another aspect of the invention there is provided a method of delivering a non-aqueous fluid comprising;
selecting a tube comprising an inner layer, an outer layer and a middle layer, wherein the inner and outer layers comprise more than about 90% by weight of an aromatic or aliphatic polyether based thermoplastic polyurethane and the middle layer comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing;
wherein the tube has a central fluid flow passage surrounded by the layers;
routing the non-aqueous fluid through the central fluid flow passage of the tube, and,
delivering the non-aqueous fluid routed through the central fluid flow passage to a preselected repository for receipt of the fluid.

In such a method the fluid preferably comprises a liquid or gaseous non-aqueous fluid selected from the group of ethers, alcohols, hydrocarbons, amines, aldehydes, ketones, amides, carboxylic acids, esters, oxygen, carbon dioxide, carbon monoxide, helium, neon, argon, krypton, xenon, radon, hydrogen and nitrogen and mixtures of two or more of the foregoing with each other or one or more of the foregoing together with an aqueous fluid.

The tube preferably does not visually delaminate at a stress of up to about 55 MPa and a strain of up to about 900-950%.

Also disclosed herein is a method of delivering a selected fluid to a selected repository comprising;
selecting a first material comprised of more than about 90% by weight of a thermoplastic polyurethane,
selecting a second material comprised of more than about 90% by weight of a thermoplastic polyurethane,
selecting a third material comprised of more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing,
forming a tube comprised of an inner layer of the first material, an outer layer of the second material and a middle layer of the third material,
wherein the middle layer adheres the inner and outer layers together;
wherein the tube has a central fluid flow passage surrounded by the inner, middle and outer layers;
routing the fluid through the central fluid flow passage of the tube, and,
delivering the fluid routed through the central fluid flow passage to the selected repository for receipt of the fluid.

In all of the embodiments and aspects of the invention described herein the polyurethane polymer or material of which the inner and outer layers are separately comprised, can be the same or can be different in structure, molecular weight, crystallinity, purity, monomeric unit content, branching, chain length, inorganic content and physical properties such as elongation, melting point, resistance to creep, and hardness.

### Brief Description of the Drawings

The drawings depict one or more embodiments of the invention that are shown by way of examples of the invention wherein:
Fig. 1 is a schematic perspective view of a tri-layered tube showing the outer and middle layers broken away in order to better illustrate the construction and arrangement of the tubing;
Fig. 2 is a cross-sectional view taken along lines 2-2 of the tube 10 shown in Fig. 1.

### Detailed Description

There is shown in FIG. 1 an embodiment of a co-extruded tri-layer tubing 10 according to the invention which comprises an outer layer 1 and an inner layer 3 each separately comprised of at least about 90% by weight of a polyurethane material, typically a polytetramethyleneglycol-based polyurethane one example of which is Lubrizol TPU Pellethane 2363-90AE. The tubing or tube 10 includes a middle layer 2 comprised of an ethylene ethyl acrylate copolymer, an ethylene methyl acrylate copolymer, an anhydride grafted ethylene methyl acrylate copolymer, a copolymer of two or more of said acrylates or a mixture of two or more of these acrylate based compounds or compositions. One example of a suitable ethylene ethyl acrylate copolymer is Dow Amplify EA 103 (Ethylene Ethyl Acrylate content being about 19.5% by weight). Examples of suitable ethylene methyl acrylate copolymers are Westlake MA SP2268 (Ethylene Methyl Acrylate content being about 24% by weight), Westlake MA SP2220 (Ethylene Methyl Acrylate content being about 20% by weight). One example of a suitable anhydride grafted ethylene methyl acrylate copolymer is Westlake Tymax GA 7001 (Anhydride grafted Ethylene Methyl Acrylate)

As shown in Fig. 1 the outer layer of polyurethane 1 has a radially inner facing surface S1 that binds and adheres to a radially outer facing surface S2 of the middle acrylate copolymer layer 2. Similarly the inner layer 3 has a radially outer facing surface S4 that binds and adheres to the radially inner facing surface S3 of the middle acrylate copolymer layer 2. The middle layer 2 adheres to the outer 1 and inner 3 layers such that the layers 1 and 3 remain adhered to layer 2 and to each other when the tube 10 is subjected to a stress of up to about 55 MPa and a strain of up to about 900-950% as measured by pulling a length of tubing 10 of about 50.8 mm (2 inches) in axial length L along its axis A using a Lloyd LR5K Plus mechanical tester at a pull rate of about 0.3048 m/minute (12 inches/minute) at ambient environmental conditions of about (22 degrees C (72 degrees F) and about 50% relative humidity, the break point of the tubing 10 being at about 57-62 MPa and at a strain about 1000-1050%. The layers 1, 2, 3 of such tubing 10 does not visually delaminate after being subjected to submersion in water at 60° C for 36 hours and subsequently mechanically flattened by manual squeezing of the tube from its normal round in cross-sectional condition to a flattened or oval shape cross-sectional shape or condition.

As shown in Figs. 1 and 2, the layers 1, 2, 3 are formed into structurally stable walls that surround and enclose a central hollow fluid passage 20 through which a fluid is routed and flows in an axial A direction contacting the radially inner facing surface S5 of the inner layer 3. The middle layer 2 binds and holds the inner 3 and outer 1 layers together.

**The** inner layer 3 provides a radially inner fluid-contact surface S5, the thickness, of the inner layer 3 typically ranging in cross-sectional thickness T1 of between about 0.00635 mm (.00025 inches) and about 0.635 mm (.025 inches). The middle layer 2 has a cross-sectional thickness T2 of between about 0.203 mm (.008 inches) and about 3.048 mm (.120 inches). The outer layer 1 typically ranges in cross-sectional thickness T3 of between about 0.00635 mm (.00025 inches) and about 0.635 mm (.025 inches).

The polyurethane elastomer (TPU) is typically the reaction product of a polyol and isocyanate and usually includes a combination of hard and soft segment domains. An aromatic polyether-based TPU or an aliphatic polyether-based TPU is used in the inner and outer layers 3, 1, such as a polytetramethyleneglycol-based polyurethane. Preferred, TPU's include the Pellethane 2363-80 AE series available from the Lubrizol Corporation, Wilmington, MA such as Lubrizol TPU Pellethane 2363-90AE.

The respective thickness of each layer of tubing 10,20 can be controlled by the extrusion tooling utilized, such as the "Tri Die" extrusion apparatus manufactured by the Genca Division of General Cable Company, Clearwater, Fla. The extrusion apparatus is selected so as to provide a uniform thickness of the layers 1, 2, 3 along the substantial entirety of the axial length L of all three layers 1, 2, 3.

The polymeric materials of which the layers 1, 2, 3 are comprised are selected so as to be visually clear or transparent and manually flexible along and around the axis A of the tubing. The polymeric materials are also selected so as to maintain the integrity of the tubing 10 (namely delamination does not occur) and its transparency or clarity after being subjected to ethylene oxide (EtO) and gamma irradiation sterilization processes.

## Claims

1. A tube (10) having a wall comprising an inner layer (3), an outer layer (1) and a middle layer (2), wherein the inner layer and outer layer each comprise more than about 90% by weight of an aromatic or aliphatic polyether based thermoplastic polyurethane and the middle layer comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

2. The tube of claim 1 wherein the middle layer comprises more than about 90% by weight of one or more of an ethylene ethyl acrylate copolymer, an ethylene methyl arcylate copolymer, or an anhydride grafted ethylene methyl acrylate copolymer, and the middle layer binds and holds together the inner and outer layers.

3. The tube of claim 2 wherein one or both of the inner and outer layers comprise a polytetramethyleneglycol-based polyurethane.

4. The tube of claim 3 wherein the ethylene ethyl acrylate copolymer comprises at least about 19.5 percent by weight ethyl acrylate content or the ethylene methyl acrylate copolymer comprises at least about 19.5 percent by weight methyl acrylate content.

5. The tube of claim 1 wherein (i) the inner and outer layers do not visually delaminate from the middle layer, at a stress up to of about 55 MPa and a strain up to about 900-950%, measured as set out in the present description, or (ii) the tube does not visually delaminate when submersed in water at 60° C for 36 hours.

6. The tube of claim 1 wherein the tube has a central axial fluid flow passage through which aqueous fluid is routed, the inner layer having a radially inner wall surface that contacts the aqueous fluid, the outer and inner layers resisting delamination from the middle layer, at a stress of up to about 55 MPa and a strain of up to about 900-950 %, measured as set out in the present description, optionally wherein the tube does not visually delaminate after being submersed in water at 60° C for 36 hours.

7. The tube of claim 1 wherein the middle layer comprises from about 5% to about 98% of the thickness of the wall or from about 10% to about 85% of the thickness of the wall or from about 61% to about 98% of the thickness of the wall.

8. The tube of claim 1 or claim 7 wherein the thickness of the middle layer is between about 0.203 mm (.008 inches) and about 3.048 mm (0.120 inches) and the thickness of each of the inner and outer layers is between about 0.00635 mm (.00025 inches) and about 0.635 mm (.025 inches).

9. Method of delivering a non-aqueous fluid comprising;
selecting a tube (10) comprising an inner layer (3), an outer layer (1) and a middle layer (2), wherein the inner layer and outer layer each comprise more than about 90% by weight of an aromatic or aliphatic polyether based thermoplastic polyurethane and the middle layer comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing;
wherein the tube has a central fluid flow passage surrounded by the layers; routing the non-aqueous fluid through the central fluid flow passage of the tube, and, delivering the non-aqueous fluid routed through the central fluid flow passage to a preselected repository for receipt of the fluid.

10. The method of claim 9 wherein the fluid comprises a liquid or gaseous non-aqueous fluid selected from the group of ethers, alcohols, hydrocarbons, amines, aldehydes, ketones, amides, carboxylic acids, esters, oxygen, carbon dioxide, carbon monoxide, helium, neon, argon, krypton, xenon, radon, hydrogen and nitrogen and mixtures of two or more of the foregoing with each other or one or more of the foregoing together with an aqueous fluid.

11. The method of claim 9, wherein (i) the tube does not visually delaminate at a stress of up to about 55 MPa and a strain of up to about 900-950%, measured as set out in the present description, (ii) the tube does not visually delaminate when submersed in water at 60° C for 36 hours.

12. The method of claim 9 wherein the middle layer comprises more than about 90% by weight of one or more of an ethylene ethyl acrylate copolymer, an ethylene methyl arcylate copolymer, or an anhydride grafted ethylene methyl acrylate copolymer, optionally wherein one or both of the inner and outer layers comprise a polytetramethyleneglycol-based polyurethane, further optionally wherein the ethylene ethyl acrylate copolymer comprises at least about 19.5 percent by weight ethyl acrylate content or the ethylene methyl acrylate copolymer comprises at least about 19.5 percent by weight methyl acrylate content.

13. The method of claim 9 wherein the inner layer has a radially inner wall surface that contacts the aqueous fluid, the outer and inner layers resisting delamination from the middle layer, at a stress of up to about 55 MPa and a strain of up to about 900-950 %, measured as set out in the present description, optionally wherein the tube does not visually delaminate after being submersed in water at 60° C for 36 hours.

14. The method of claim 9 wherein the middle layer comprises from about 5% to about 98% of the thickness of the wall or from about 10% to about 85% of the thickness of the wall or from about 61% to about 98% of the thickness of the wall.

15. The method of claim 9 or claim 14 wherein the thickness of the middle layer is between about 0.203 mm (.008 inches) and about 3.048 mm (0.120 inches) and the thickness of each of the inner and outer layers is between about 0.00635 mm (.00025 inches) and about 0.635 mm (.025 inches).

## Patentansprüche

1. Rohr (10) mit einer Wand, die eine innere Schicht (3), eine äußere Schicht (1) und eine mittlere Schicht (2) aufweist, wobei die innere Schicht und die äußere Schicht jeweils mehr als etwa 90 Gew.-% eines thermoplastischen Polyurethans auf Basis eines aromatischen oder aliphatischen Polyethers aufweisen und die mittlere Schicht ein Ethylen-Ethylacrylat-Copolymer oder ein Ethylen-Methylacrylat-Copolymer oder ein Anhydrid-gepfropftes Ethylen-Methylacrylat-Copolymer, ein Copolymer aus zwei oder mehr dieser Acrylate oder eine Mischung aus zwei oder mehr der vorgenannten aufweist.

2. Rohr nach Anspruch 1, bei welchem die mittlere Schicht mehr als etwa 90 Gew.-% eines oder mehrerer von einem Ethylen-Ethylacrylat-Copolymer, einem Ethylen-Methyl-Arcylat-Copolymer oder einem Anhydrid-gepfropften Ethylen-Methylacrylat-Copolymer aufweist und die mittlere Schicht die innere und die äußere Schicht bindet und zusammenhält.

3. Rohr nach Anspruch 2, bei welchem eine oder beide der inneren und der äußeren Schicht ein Polyurethan auf Polytetramethylenglykolbasis aufweisen.

4. Rohr nach Anspruch 3, bei welchem das Ethylen-Ethylacrylat-Copolymer mindestens etwa 19,5 Gew.-% Ethylacrylatgehalt aufweist oder das Ethylen-Methylacrylat-Copolymer mindestens etwa 19,5 Gew.-% Methylacrylatgehalt aufweist.

5. Rohr nach Anspruch 1, bei welchem (i) die innere und die äußere Schicht bei einer Spannung ("stress") von bis zu etwa 55 MPa und einer Dehnung ("strain") von bis zu etwa 900-950%, gemessen wie in der vorliegenden Beschreibung dargelegt, nicht sichtbar von der mittleren Schicht delaminieren oder (ii) das Rohr nicht sichtbar delaminiert, wenn es für 36 Stunden in Wasser bei 60°C untergetaucht wird.

6. Rohr nach Anspruch 1, bei welchem das Rohr einen zentralen axialen Fluidströmungskanal aufweist, durch den ein wässriges Fluid geleitet wird, wobei die innere Schicht eine radial innere Wandfläche hat, die das wässrige Fluid kontaktiert, wobei sich die äußere und die innere Schicht einer Delaminierung von der mittleren Schicht bei einer Spannung ("stress") von bis zu etwa 55 MPa und einer Dehnung ("strain") von bis zu etwa 900-950 %, gemessen wie in der vorliegenden Beschreibung dargelegt, widersetzen, wobei optional das Rohr nicht sichtbar delaminiert, nachdem es für 36 Stunden in Wasser bei 60°C untergetaucht war.

7. Rohr nach Anspruch 1, bei welchem die mittlere Schicht etwa 5% bis etwa 98% der Wanddicke oder etwa 10% bis etwa 85% der Wanddicke oder etwa 61% bis etwa 98% der Wanddicke ausmacht.

8. Rohr nach Anspruch 1 oder Anspruch 7, bei welchem die Dicke der mittleren Schicht zwischen etwa 0,203 mm (0,008 Zoll) und etwa 3,048 mm (0,120 Zoll) liegt und die Dicke jeder der inneren und der äußeren Schicht zwischen etwa 0,00635 mm (0,00025 Zoll) und etwa 0,635 mm (0,025 Zoll) liegt.

9. Verfahren zum Fördern eines nichtwässrigen Fluids, aufweisend:
Auswählen eines Rohrs (10) mit einer inneren Schicht (3), einer äußeren Schicht (1) und einer mittleren Schicht (2), wobei die innere Schicht und die äußere Schicht jeweils mehr als etwa 90 Gew.-% eines thermoplastischen Polyurethans auf Basis eines aromatischen oder aliphatischen Polyethers aufweisen und die mittlere Schicht ein Ethylen-Ethylacrylat-Copolymer oder ein Ethylen-Methylacrylat-Copolymer oder ein Anhydrid-gepfropftes Ethylen-Methylacrylat-Copolymer, ein Copolymer aus zwei oder mehr dieser Acrylate oder eine Mischung aus zwei oder mehr der vorgenannten aufweist;
wobei das Rohr einen zentralen Fluidströmungskanal aufweist, der von den Schichten umgeben ist;
Leiten des nichtwässrigen Fluids durch den zentralen Fluidströmungskanal des Rohrs, und
Übergeben des nichtwässrigen Fluids, das durch den zentralen Fluidströmungskanal geleitet wird, an eine vorausgewählte Lagerstätte zur Aufnahme des Fluids.

10. Verfahren nach Anspruch 9, bei welchem das Fluid ein flüssiges oder gasförmiges nichtwässriges Fluid, ausgewählt aus der Gruppe von Ethern, Alkoholen, Kohlenwasserstoffen, Aminen, Aldehyden, Ketonen, Amiden, Karbonsäuren, Estern, Sauerstoff, Kohlendioxid, Kohlenmonoxid, Helium, Neon, Argon, Krypton, Xenon, Radon, Wasserstoff und Stickstoff und Mischungen von zwei oder mehr der vorgenannten miteinander oder einer oder mehreren der vorgenannten zusammen mit einem wässrigen Fluid, aufweist.

11. Verfahren nach Anspruch 9, bei welchem (i) das Rohr sich bei einer Spannung ("stress") von bis zu etwa 55 MPa und einer Dehnung ("strain") von bis zu etwa 900-950%, gemessen wie in der vorliegenden Beschreibung dargelegt, nicht sichtbar delaminiert, (ii) das Rohr nicht sichtbar delaminiert, wenn es für 36 Stunden in Wasser bei 60°C untergetaucht wird.

12. Verfahren nach Anspruch 9, bei welchem die mittlere Schicht mehr als etwa 90 Gew.-% einer oder mehrerer von einem Ethylen-Ethylacrylat-Copolymer, einem Ethylen-Methyl-Arcylat-Copolymer oder einem Anhydrid-gepfropften Ethylen-Methylacrylat-Copolymer aufweist, und bei welchem optional eine oder beide der inneren und der äußeren Schicht ein Polyurethan auf Polytetramethylenglykolbasis aufweisen, wobei ferner optional das Ethylen-Ethylacrylat-Copolymer mindestens etwa 19,5 Gew.-% Ethylacrylatgehalt aufweist oder das Ethylen-Methylacrylat-Copolymer mindestens etwa 19,5 Gew.-% Methylacrylatgehalt aufweist.

13. Verfahren nach Anspruch 9, bei welchem die innere Schicht eine radial innere Wandfläche aufweist, die das wässrige Fluid kontaktiert, wobei sich die äußere und die innere Schicht einer Delaminierung von der mittleren Schicht bei einer Spannung ("stress") von bis zu etwa 55 MPa und einer Dehnung ("strain") von bis zu etwa 900-950 %, gemessen wie in der vorliegenden Beschreibung dargelegt, widersetzen, wobei optional das Rohr nicht sichtbar delaminiert, nachdem es für 36 Stunden in Wasser bei 60°C untergetaucht war.

14. Verfahren nach Anspruch 9, bei welchem die mittlere Schicht etwa 5% bis etwa 98% der Wanddicke oder etwa 10% bis etwa 85% der Wanddicke oder etwa 61% bis etwa 98% der Wanddicke ausmacht.

15. Verfahren nach Anspruch 9 oder Anspruch 14, bei welchem die Dicke der mittleren Schicht zwischen etwa 0,203 mm (0,008 Zoll) und etwa 3,048 mm (0,120 Zoll) liegt und die Dicke jeder der inneren und der äußeren Schicht zwischen etwa 0,00635 mm (0,00025 Zoll) und etwa 0,635 mm (0,025 Zoll) liegt.

## Revendications

1. Tube (10) ayant une paroi comprenant une couche intérieure (3), une couche extérieure (1) et une couche médiane (2), dans lequel la couche intérieure et la couche extérieure comprennent chacune plus de 90 % environ en poids d'un polyuréthane thermoplastique à base de polyéther aromatique ou aliphatique, et dans lequel la couche médiane comprend un copolymère éthylène - acrylate d'éthyle ou un copolymère éthylène - acrylate de méthyle ou un copolymère éthylène - acrylate de méthyle greffé anhydride, un copolymère de deux ou plusieurs desdits acrylates ou un mélange de deux ou plusieurs de ce qui précède.

2. Tube selon la revendication 1, dans lequel la couche médiane comprend plus de 90 % environ en poids d'un ou plusieurs parmi un copolymère éthylène - acrylate d'éthyle, un copolymère éthylène - acrylate de méthyle ou un copolymère éthylène - acrylate de méthyle greffé anhydride, et dans lequel la couche médiane lie et maintient ensemble les couches intérieure et extérieure.

3. Tube selon la revendication 2, dans lequel l'une ou les deux parmi les couches intérieure et extérieure comprennent un polyuréthane à base de polytétraméthylèneglycol.

4. Tube selon la revendication 3, dans lequel le copolymère éthylène - acrylate d'éthyle comprend au moins 19,5 % environ en poids de teneur en acrylate d'éthyle ou le copolymère éthylène - acrylate de méthyle comprend au moins 19,5 % environ en poids de teneur en acrylate de méthyle.

5. Tube selon la revendication 1, dans lequel (i) les couches intérieure et extérieure ne se délaminent pas visuellement à partir de la couche médiane sous une contrainte allant jusqu'à 55 MPa environ et une déformation allant jusqu'à 900 - 950 % environ, ceci mesuré comme indiqué dans la présente description, ou dans lequel (ii) le tube ne se délamine pas visuellement lorsqu'il est immergé dans l'eau à 60 °C pendant 36 heures.

6. Tube selon la revendication 1, dans lequel le tube a un passage d'écoulement de fluide axial central à travers lequel un fluide aqueux est acheminé, la couche intérieure ayant une surface de paroi radialement intérieure qui est en contact avec le fluide aqueux, les couches intérieure et extérieure résistant au délaminage à partir de la couche médiane sous une contrainte allant jusqu'à 55 MPa environ et une déformation allant jusqu'à 900 - 950 % environ, ceci mesuré comme indiqué dans la présente description, éventuellement dans lequel le tube ne se délamine pas visuellement après avoir été immergé dans l'eau à 60 °C pendant 36 heures.

7. Tube selon la revendication 1, dans lequel la couche médiane comprend entre 5 % environ et 98 % environ de l'épaisseur de la paroi ou entre 10 % environ et 85 % environ de l'épaisseur de la paroi ou entre 61 % environ et 98 % environ de l'épaisseur de la paroi.

8. Tube selon la revendication 1 ou 7, dans lequel l'épaisseur de la couche médiane est comprise entre 0,203 mm (0,008 pouce) environ et 3,048 mm (0,120 pouce) environ, et dans lequel l'épaisseur de chacune des couches intérieure et extérieure est comprise entre 0,00635 mm (0,00025 pouce) environ et 0,635 mm (0,025 pouce) environ.

9. Procédé de délivrance d'un fluide non aqueux consistant à :
sélectionner un tube (10) comprenant une couche intérieure (3), une couche extérieure (1) et une couche médiane (2), dans lequel la couche intérieure et la couche extérieure comprennent chacune plus de 90 % environ en poids d'un polyuréthane thermoplastique à base de polyéther aromatique ou aliphatique, et dans lequel la couche médiane comprend un copolymère éthylène - acrylate d'éthyle ou un copolymère éthylène - acrylate de méthyle ou un copolymère éthylène - acrylate de méthyle greffé anhydride, un copolymère de deux ou plusieurs desdits acrylates ou un mélange de deux ou plusieurs de ce qui précède ;
dans lequel le tube a un passage d'écoulement de fluide central entouré des couches ;
acheminer le fluide non aqueux à travers le passage d'écoulement de fluide central du tube, et
délivrer le fluide non aqueux acheminé à travers le passage d'écoulement de fluide central à un endroit de dépôt présélectionné pour la réception du fluide.

10. Procédé selon la revendication 9, dans lequel le fluide comprend un fluide non aqueux liquide ou gazeux choisi dans le groupe des éthers, des alcools, des hydrocarbures, des aminés, des aldéhydes, des cétones, des amides, des acides carboxyliques, des esters, de l'oxygène, du dioxyde de carbone, du monoxyde de carbone, de l'hélium, du néon, de l'argon, du krypton, du xénon, du radon, de l'hydrogène, de l'azote et des mélanges de deux ou plusieurs de ce qui précède avec les uns et les autres ou d'un ou plusieurs de ce qui précède ensemble avec un fluide aqueux.

11. Procédé selon la revendication 9, dans lequel (i) le tube ne se délamine pas visuellement sous une contrainte allant jusqu'à 55 MPa environ et une déformation allant jusqu'à 900 - 950 % environ, ceci mesuré comme indiqué dans la présente description, ou dans lequel (ii) le tube ne se délamine pas visuellement lorsqu'il est immergé dans l'eau à 60 °C pendant 36 heures.

12. Procédé selon la revendication 9, dans lequel la couche médiane comprend plus de 90 % environ en poids d'un ou plusieurs parmi un copolymère éthylène - acrylate d'éthyle, un copolymère éthylène - acrylate de méthyle ou un copolymère éthylène - acrylate de méthyle greffé anhydride, éventuellement dans lequel l'une ou les deux parmi les couches intérieure et extérieure comprennent un polyuréthane à base de polytétraméthylèneglycol, en outre éventuellement dans lequel le copolymère éthylène - acrylate d'éthyle comprend au moins 19,5 % environ en poids de teneur en acrylate d'éthyle ou le copolymère éthylène - acrylate de méthyle comprend au moins 19,5 % environ en poids de teneur en acrylate de méthyle.

13. Procédé selon la revendication 9, dans lequel la couche intérieure a une surface de paroi radialement intérieure qui est en contact avec le fluide aqueux, les couches intérieure et extérieure résistant au délaminage à partir de la couche médiane sous une contrainte allant jusqu'à 55 MPa environ et une déformation allant jusqu'à 900 - 950 % environ, ceci mesuré comme indiqué dans la présente description, éventuellement dans lequel le tube ne se délamine pas visuellement après avoir été immergé dans l'eau à 60 °C pendant 36 heures.

14. Procédé selon la revendication 9, dans lequel la couche médiane comprend entre 5 % environ et 98 % environ de l'épaisseur de la paroi ou entre 10 % environ et 85 % environ de l'épaisseur de la paroi ou entre 61 % environ et 98 % environ de l'épaisseur de la paroi.

15. Procédé selon la revendication 9 ou 14, dans lequel l'épaisseur de la couche médiane est comprise entre 0,203 mm (0,008 pouce) environ et 3,048 mm (0,120 pouce) environ, et dans lequel l'épaisseur de chacune des couches intérieure et extérieure est comprise entre 0,00635 mm (0,00025 pouce) environ et 0,635 mm (0,025 pouce) environ.
